# EUROPEAN PATENT APPLICATION

(11) **EP 3 660 859 A1**
(43) Date of publication of application: **03.06.2020**
(21) Application number: 18209068.8
(22) Date of filing: 29.11.2018
(51) Int. Cl.: G16H 40/20, G16H 50/30

(54) **INTELLIGENT AUTONOMOUS PATIENT ROUTING FOR SCANS**

(71) Applicant: Koninklijke Philips N.V., 5656 AG Eindhoven (NL)
(72) Inventor: SISODIA, Rajendra Singh, 5656 AE Eindhoven (NL); VUPPALA, Sunil Kumar, 5656 AE Eindhoven (NL); CHAUDHURY, Sudipta, 5656 AE Eindhoven (NL); BHAT, Ravindra, 5656 AE Eindhoven (NL); JOHNSON, Mark Thomas, 5656 AE Eindhoven (NL)
(74) Representative: de Haan, Poul Erik

(57) **Abstract**

The present invention relates to patient routing. In order to provide a more efficient patient flow for autonomous image acquisition, a method is provided for controlling a patient transfer apparatus within a healthcare facility. The method comprises the following steps: providing a routing information for transferring the patient transfer apparatus to a required location; controlling the patient transfer apparatus to move on a first guided path according to the provided routing information; detecting at least one of a vital sign and an abnormal movement of the patient during a transit of the patient transfer apparatus; evaluating a patient condition based on at least one of the detected vital sign and the detected abnormal movement of the patient; updating the routing information in response to the evaluated patient condition, if the evaluated patient condition meets a predefined criterion; and controlling the patient transfer apparatus to move on a second guided path according to the updated routing information during the transit of the patient transfer apparatus.

## Description

### FIELD OF THE INVENTION

The present invention relates to patient routing. In particular, the present invention relates to a method and a system for controlling a patient transfer apparatus within a healthcare facility.

### BACKGROUND OF THE INVENTION

Indoor routing of a patient within a healthcare facility, e.g. within a hospital, may be time consuming and may involve manually intensive tasks. In-patients are usually routed (or transferred) on a bed being pulled or pushed by a nursing support staff. Motorized beds can automatically move a patient based on the conditions of surroundings.

US 2014/095011 A1 describes an automated device for moving a patient to and from various locations, care units, etc., within a care facility.

### SUMMARY OF THE INVENTION

There may be a need to provide a more efficient patient flow for autonomous image acquisition.

The object of the present invention is solved by the subject-matter of the independent claims, wherein further embodiments are incorporated in the dependent claims. It should be noted that the following described aspects of the invention apply also for the method and for the system.

A first aspect of the invention relates to a method for controlling a patient transfer apparatus within a healthcare facility. The method comprises the steps of providing a routing information for transferring the patient transfer apparatus to a required location; controlling the patient transfer apparatus to move on a first guided path according to the provided routing information; detecting at least one of a vital sign and an abnormal movement of the patient during a transit of the patient transfer apparatus; evaluating a patient condition based on at least one of the detected vital sign and the detected abnormal movement of the patient; updating the routing information in response to the evaluated patient condition, if the evaluated patient condition meets a predefined criterion; and controlling the patient transfer apparatus to move on a second guided path according to the updated routing information during the transit of the patient transfer apparatus.

In other words, a method is provided for automatically providing routing information to a patient transport apparatus, e.g. a bed or an autonomous movement unit. The routing information may be displayed on the patient transport apparatus to be moved, but can also be used for routing. The patient transfer apparatus will consider the patient condition during routing. Accordingly, the patient transfer apparatus may route and move the patient to a different location, if the patient condition changes.

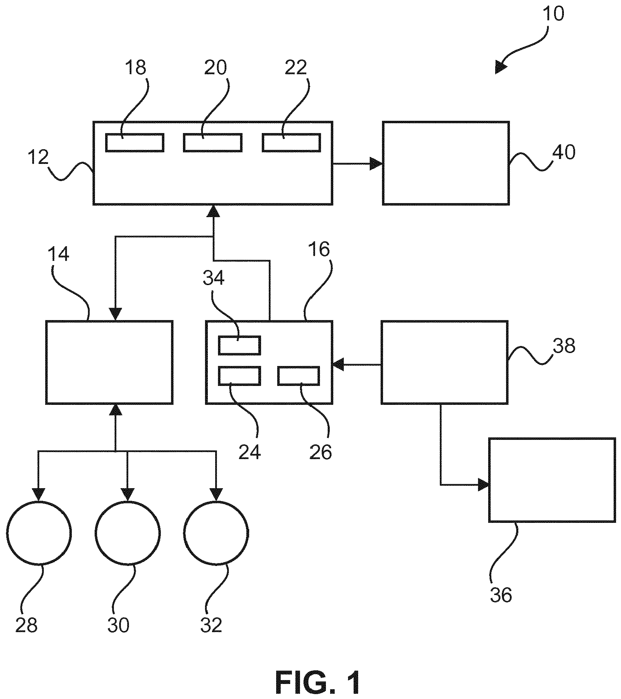

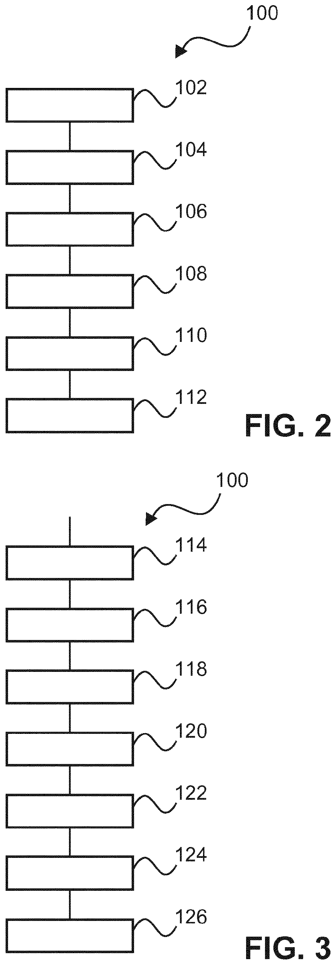

As will be explained hereafter and particularly with respect to the exemplary embodiment of Figs. 1 and 2, the patient transfer apparatus may determine the relative priority and right of way dynamically. Further, the integration with a centralized scheduling system will also provide accurate information about patient movement, time, duration, slots, etc., using two-way communication between a scan unit, a centralized scheduling system and the patient transfer apparatus.

The "patient transfer apparatus" as used herein may refer to an apparatus, such as a bed, a wheelchair, or an autonomous movement unit, for transferring a patient from one location to another within a healthcare facility. The patient transfer apparatus may be provided with a patient support system, such as connected monitors, sensors, residual devices strapped, e.g. drugs, saline, etc. The patient transfer apparatus may further comprise a display for visualizing the routing information as a road map to assist a user with a manual decision.

The "routing information" as used herein may refer to a route map within the healthcare facility for moving the patient transfer apparatus from one location to another. In some implementations, the routing information may be based on patient, type of scan, time and modality type. For example, the routing information may comprise the estimation of the right route, time of movement and approach based on the type of scan and modality. The routing information may be displayed, visualized, e.g. on a display attached to the patient transfer apparatus, to provide required information.

The "patient condition" as used herein may refer to the patient's current state, such as being good or serious. The "patient condition" may also refer to the patient short-term prognosis, for example, the patient is improving, is getting worse, or no immediate change is expected, i.e. stable. The patient condition may be evaluated and categorized in the following terms: undetermined (patient awaiting physician and/or assessment), good (vital signs are stable and within normal limits), serious (serious vital signs may be unstable and not within normal limits), and critical (critical vital signs are unstable and not within normal limits). The patient condition may be monitored and evaluated continuously, e.g. every 10 seconds, 50 seconds, 100 seconds, or another time interval.

The "updating the routing information" as used herein may refer to changing the routing information, if the evaluated patient condition meets a predefined criterion. For example, the predefined criterion may be a downgrade of the patient condition, including the current status of the patient and short-term prognosis, such as a downgrade from "good" to "serious". This usually indicates that a patient is likely to be in the intensive care unit (ICU) or acute ward. If this predefined criterion is met, the patient transfer apparatus will be controlled to move the patient to the ICU for doctor's attention. The routing information may also be changed by an external event, e.g. if the slot allocated to the patient is provided to another emergency/acute patient.

The "second guided path" is different from the "first guided path".

According to an embodiment of the invention, the method further comprises the step of providing a priority information for triggering a control requirement for an access to a common facility within the healthcare facility such that when the patient transfer apparatus arrives at the common facility, the patient transfer apparatus is given a priority access to the common facility. Alternatively or additionally, the method further comprises the steps of providing a priority information for determining a relative right of way for two or more patient transfer apparatuses; determining, according to the priority information, a first relative right of way of the patient transfer apparatus, when another patient transfer apparatus is encountered; controlling the patient transfer apparatus to move according to the first relative right of way; updating the priority information in response to the evaluated patient condition, if the evaluated patient condition meets the predefined criterion; determining, according to the updated priority information, a second relative right of way of the patient transfer apparatus, when another patient transfer apparatus is encountered; and controlling the patient transfer apparatus to move according to the second relative right of way.

In other words, the patient transfer apparatus will consider the patient condition during routing and determine the relative priority and right of way dynamically such that a patient with a serious patient condition will be transferred with precedence over others. This may include the priority for the access to common facilities, such as lift, access to corridors, so that when the patient transfer apparatus arrives, they may be at right location (floor) or reserved for them as well. This will be explained hereafter and particularly with respect to the exemplary embodiment of Figs. 1 and 3.

The "priority information", or "right or way", as used herein may refer to the right of the patient transfer apparatus to proceed with precedence over others in a particular situation or place within the healthcare facility. The priority of routing may be required when a patient condition is or is becoming serious and thus the patient transfer apparatus carrying that patient has the right of way. The priority of routing may also be required to use common block points, e.g. the usage of lifts, corridors.

The "updating the priority information" as used herein may refer to changing the priority information, if the evaluated patient condition meets the predefined criterion.

According to an embodiment of the invention, the priority information is based on a decision from a local distributed system. In the local distributed system, each patient transfer apparatus is configured to negotiate and discuss with other patient transfer apparatuses according to a predefined protocol to determine each other's right or way. Alternatively, the priority information is based on a decision from a centrally coordinated system. In the centrally coordinated system, the routing information of each patient transfer apparatus and the patient condition of the patient on each patient transfer apparatus are configured to be updated to determine the priority information.

According to an embodiment of the invention, the method further comprises the steps of providing a scan schedule to trigger a movement of the patient transfer apparatus; determining the routing information based on the provided scan schedule; controlling the patient transfer apparatus to move on a first guided path according to the determined routing information; updating the scan schedule in response to the evaluated patient condition, if the evaluated patient condition meets the predefined criterion; updating the routing information and/or the priority information in response to the updated scan schedule; and controlling the patient transfer apparatus to move on a second guided path according to the updated routing information and/or the updated priority information during the transit of the patient transfer apparatus.

Based on the scan schedule, such as the availability of the scanner, the type of scan and modality, the right route, time of movement and approach, can be estimated for determining the routing information and/or priority information. This will be explained hereafter and particularly with respect to the exemplary embodiment of Figs. 1 and 3.

For example, the patient transfer apparatus may be linked with a central scan schedule system to determine when the next patient is due. The duration of the patient movement during the routing may be estimated. The type of scan and modality may also be required to estimate the right route, time of movement and approach.

The "scan schedule" as used herein may refer to the schedules of the patient for the scans with one or more patient scan systems. The scan schedule may determine when the next patient is due. The scan schedule may comprise the availability of a scanner, a scan type, a scan duration, a modality type and/or a scheduling of scan slot.

The "updating the scan schedule" as used herein may refer to changing the scan schedule, if the evaluated patient condition meets a predefined criterion.

According to an embodiment of the invention, the method further comprises obtaining a local routing information of the patient transfer apparatus during transit, and updating the scan schedule of the patient based on the local routing information of the patient transfer apparatus.

The coordination between the central scan schedule system and the routing device of individual patient transfer apparatus may allow a dynamic change in the scan schedule based on the patient condition and a change in clinical pathways.

The "local routing information" as used herein may refer to the routing information relating to the surroundings of the patient transfer apparatus. The local routing information may comprise position information of the patient transfer apparatus, traffic conditions of the surroundings of the patient transfer apparatus, etc.

According to an embodiment of the invention, the scan schedule comprises at least one of the following: availability of a scanner, a scan type, a scan duration, a modality type, and a scheduling of scan slot.

The scan schedule may be used to trigger the movement of the patient transfer apparatus

The availability of the scanner may indicate when the next patient is due.

The scan type may comprise magnetic resonance imaging (MRI), computed tomography (CT), positron emission tomography (PET), X-ray, ultrasound, mammography, nuclear imaging, etc.

The scan duration may indicate how long the scan last. For example, the CT scan duration only lasts between 5 and 10 minutes to perform, and that includes preparation time.

According to an embodiment of the invention, the method further comprises visualizing the routing information as a road map to assist a user with a manual decision.

The routing device may be configured to download the route map for a patient in an autonomous scan system. This map can also be displayed, visualized on a display attached to the patient transfer apparatus to provide required information.

According to an embodiment of the invention, the patient condition comprises at least one of the following: a current status of the patient based on an evaluation of at least one of the detected vital sign and the detected abnormal movement of the patient, and a patient's short-term prognosis based on an evaluation of at least one of the detected vital sign and the at least one detected abnormal movement of the patient, and historical data of the patient or similar patients.

The "patient's current state" maybe reported, e.g., as being good or serious.

The "patient's short-term prognosis" may be reported, e.g., that the patient is improving, is getting worse, or that no immediate change is expected, i.e. stable.

According to an embodiment of the invention, a predicted model is provided to evaluate the patient's short-term prognosis. The predicted model comprises at least one of the following: a deep learning model, and a statistical model.

In an example, the statistical model is an auto regressive integrated moving average (ARIMA) model.

In an example, the deep learning model is a long short-term memory (LSTM) model.

A second aspect of the invention relates to a system for controlling a patient transfer apparatus within a healthcare facility. The system comprises: i) a patient transport apparatus, ii) an in-transit patient condition assessor, and iii) a clinical support subsystem. The patient transport apparatus comprises a drive device, a control device, and a routing device. The clinical support subsystem comprises a routing planning device and a patient condition evaluation device. The route-planning device is configured to provide a routing information to the routing device of the patient transfer apparatus for transferring the patient transfer apparatus to a required location. The drive device is configured to be controlled by the control device to move the patient transfer apparatus on a first guide path according to the routing information received by the routing device. The in-transit patient condition assessor is configured to detect at least one of a vital sign and an abnormal movement of the patient during a transit of the patient transfer apparatus. The patient condition evaluation device of the clinical decision support system is configured to evaluate a patient condition based on at least one of the detected vital sign and the detected abnormal movement of the patient. The route-planning device is configured to update the routing information in response to the evaluated patient condition, if the evaluated patient condition meets a predefined criterion. The control device of the patient transfer apparatus is configured to control the drive device to move the patient transfer apparatus on a second guided path according to the updated routing information during the transit of the patient transfer apparatus.

The "drive device" as used herein may refer to a device to cause the patient transfer apparatus to move. For example, the drive device may include electric motors and wheels that are driven by the electric motors and can move in all the front, back, right, and left directions.

The "control device" as used herein may refer to a data processing element such as a microprocessor, microcontroller, field programmable gate array (FPGA), central processing unit (CPU), digital signal processor (DSP) capable of providing a control signal to the drive device via the universal service bus (USB), copper wires, optical fibres, or another form of data connection.

The "routing device" may refer to a guiding device, which may be implemented as a data processing element, capable of receiving routing information e.g. via a local area network connection (LAN) and providing the routing information to the control device, e.g. via a physical cable.

The "patient condition evaluation device" as used herein may refer to a data processing element capable of receiving the detected vital sign and the detected abnormal movement, and/or historical data of the patient or similar patients, and evaluating the patient condition based thereon. The short-term prognosis may be evaluated with a predicted model, such as a supported machine-learning model or a deep learning model, built on historical data. The predicted model is not limited to statistical based techniques, such as ARIMA, but extended to LSTM type of deep learning models if there are sufficient data of the patient or similar patients.

The "route-planning device" as used herein may refer to a data processing element capable of receiving the patient condition output from the patient condition evaluation device and updating the routing information accordingly.

The patient condition evaluation device of the clinical decision support system is configured to evaluate a patient condition based on at least one of the detected vital sign and the detected abnormal movement of the patient, and historical data of the patient and/or similar patients based on a predicted model. The predicted model comprises at least one of the following: a deep learning model, and a statistical model.

According to an embodiment of the invention, the in-transit patient condition assessor comprises at least one of the following: a camera configured to detect an abnormal movement of the patient, a touchless sensor arranged on the patient transfer apparatus and configured to detect a vital sign and/or an abnormal movement, and a patient support system arranged on the patient transfer apparatus and configured to detect a vital sign.

The patient support system may comprise connected monitors, sensors, and residual devices strapped.

In hospitals, cameras are already provided in corridors and lifts for safety or surveillance purposes. These cameras may also be used for vital sign monitoring and/or abnormal movements of patients. This may be used to alert the routing system or the concerned human observer for prioritized route or movements.

According to an embodiment of the invention, the system further comprises a priority-planning device configured to provide a priority information for triggering a control requirement for an access to a common facility within the healthcare facility such that when the patient transfer apparatus arrives at the common facility, the patient transfer apparatus is given a priority access to the common facility. Alternatively or additionally, the priority-planning device is configured to provide a priority information for determining a relative right of way for two or more patient transfer apparatuses. The priority-planning device is configured to determine, according to the priority information, a first relative right of way of the patient transfer apparatus, when another patient transfer apparatus is encountered. The control device of the patient transfer apparatus is configured to control the drive device to move the patient transfer apparatus according to the first relative right of way. The priority-planning device is configured to update the priority information in response to the valuated patient condition, if the evaluated patient condition meets the predefined criterion. The priority-planning device is configured to determine, according to the updated priority information, a second relative right of way of the patient transfer apparatus, when another patient transfer apparatus is encountered. The control device of the patient transfer apparatus is configured to control the drive device to move the patient transfer apparatus according to the second relative right of way.

In other words, the priority-planning device may also trigger specific control requirements, for e.g. access to common facilities such as lift, access to corridors, so that when the patient transfer apparatus arrives, they may be at right location (floor) or reserved for them as well.

The priority-planning device may be provided as a device of the patient transfer apparatus for providing the priority information based on a decision from a local distributed system. In the local distributed system, the priority-planning device of each patient transfer apparatus is configured to negotiate and discuss with that of other patient transfer apparatuses according to a predefined protocol to determine each other's right or way.

Alternatively and additionally, the priority-planning device may be provided as a device of the clinical decision support subsystem for providing the priority information based on a decision from a centrally coordinated system. In in the centrally coordinated system, the routing information of each patient transfer apparatus and the patient condition of the patient on each patient transfer apparatus are configured to be updated to determine the priority information with the priority-planning device of the clinical decision support subsystem.

According to an embodiment of the invention, the system further comprises at least one scanner, and a central scan schedule subsystem. The at least one scanner is configured to perform medical imaging examinations on the patient. The central scan schedule subsystem is configured to coordinate with the at least one scanner to provide a scan schedule of the patient for triggering a movement of the patient transfer apparatus towards the at least one scanner. The priority-planning device of the clinical decision support subsystem is configured to determining the routing information based on the provided scan schedule. In the control device of the patient transfer apparatus is configured to control the drive device to move the patient transfer apparatus on a first guided path according to the determined routing information. The central scan schedule subsystem is configured to update the scan schedule in response to the evaluated patient condition, if the evaluated patient condition meets the predefined criterion. The route-planning device and/or the priority-planning device are configured to update the routing information and/or the priority information in response to the updated scan schedule. The control device of the patient transfer apparatus is configured to control the drive device to move the patient transfer apparatus on a second guided path according to the updated routing information and/or the updated priority information during the transit of the patient transfer apparatus.

In other words, the central scan schedule subsystem may be configured to coordinate with the routing device of the patient transfer apparatus to obtain a local routing information of the patient transfer apparatus during transit. The central scan schedule subsystem is configured to update the scan schedule of the patient based on the local routing information.

The system may comprise multiple scanners of different modalities, such as an MRI scanner, a CT scanner, a PET scanner, etc.

An autonomous medical imaging system may also be part of the present invention. The autonomous medical imaging system comprises an autonomous scanner, and a system for controlling a patient transfer apparatus as described above and below. The patient transfer apparatus is configured to transfer the patient in and out of the autonomous scanner and to position at a desired location for medical imaging. The autonomous scanner is configured to have a scan of the patient when the patient support is positioned at the desired location to acquire a medical image.

The autonomous scanner may be an autonomous CT scanner, an autonomous MRI scanner, an autonomous PET scanner, etc.

According to an aspect of the invention, a method is provided for automatically providing routing information to a patent transfer apparatus, such as a bed or an autonomous movement unit. The routing information may be displayed on a bed to be moved but also may be used for routing.

In an example, the patient transfer apparatus will take patient condition during routing and determine the relative priority and right of way dynamically. For example, One solution is to use data from patient support system, such as connected monitors, sensors, residual devices strapped (drugs, saline, etc.) to decide on criticality, change in conditions, priority, routes to take, and routes to avoid, etc. The calculation of criticality can be based on an evaluation of physiological parameters by using a clinical decision support system. The change in patient condition will cause the patient transfer apparatus to take that patient to a required location. The clinical decision support system has various components such as predication of patient condition, route recommendation with supported machine learning or deep learning models built on historical data. It may use historical data of the patient to predict the patient condition and criticality with the predicted models. The predicted models are not limited to statistical based techniques such as ARIMA, but extended to LSTM type of deep learning models, if there are sufficient data of the patient/similar patients.

In an example, a priority of the system is to determine the relative right of way for two autonomous systems. Route specific preferences are based on whether the current bed is compatible to specific modality, attachments to the patients, etc. The change in patient condition results in change in the priority and the route to follow. These changes are coordinated with a central scan schedule system as it effects the overall scheduling of patients for the scans. For instance, if the patient is not ready for the scan due to the change in his clinical parameters during the transit to the scan room, the system routes the patient to ICU (Intensive Care Unit) for doctor's attention immediately and updates the same to the central scan schedule system so that the reschedule of scan is performed in real time. This improves the overall efficiency of the system in scheduling the patients for the scans and routing them autonomously. Based on the patient condition, the system suggests the routes to take and routes to avoid. This information is also updated based on the route taken by each patient bed to the scan. This continuous update of the information is also fed to the route recommendation system of clinical decision support system.

In an example, the priority of routing is required for e.g. to use common block points, such as the usage of lifts, corridors. Further, it is also to determine which patient movement is prioritized over others. The autonomous routing system can decide the relative priority of patient routing either based on a local distributed system or a central coordinated system. In a centrally coordinated system, the routing information for each system and patient health information is dynamically updated to determine the relative priority and routing information. In a distributed system, each autonomous patient routing system negotiates and discuss with other system as per defined protocol to determine each other's right of way. Each method has its own advantages and disadvantages in terms of scalability, operation and computational requirements.

In an example, the system is linked with central scan schedule system to determine when the next patient is due. The route estimation decides the duration of patient movement during the routing. Further, the type of scan and modality is also required to estimate the right route, time of movement and approach. The coordination between central scan schedule system and individual patient routing systems allows doing the dynamic changes in the schedules based on the patient condition and change in clinical pathways.

In an example, the system may have an ability to download a route map for a patient in an autonomous scan system. The routing information will be based on patient, type of scan, time and modality type. This map can also be displayed, visualized on the display attached to the system so as to provide required information.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic diagram of a system for controlling a patient transfer apparatus within a healthcare facility according to an embodiment of the invention.
Fig. 2 shows a schematic diagram of a method for controlling a patient transfer apparatus within a healthcare facility according to an embodiment of the invention.
Fig. 3 shows a schematic diagram of a method for controlling a patient transfer apparatus within a healthcare facility according to a further embodiment of the invention.
Fig. 4 shows a schematic diagram of a method for controlling a patient transfer apparatus within a healthcare facility according to a further embodiment of the invention.
Fig. 5 shows a schematic diagram of a method for controlling a patient transfer apparatus within a healthcare facility according to a further embodiment of the invention.

### DETAILED DESCRIPTION OF EMBODIMENTS

Fig. 1 shows a system 10 for controlling a patient transfer apparatus within a healthcare facility according to an embodiment of the invention. The system 10 comprises a patient transport apparatus 12, an in-transit patient condition assessor 14, and a clinical support subsystem 16. The patient transport apparatus 12 comprises a drive device 18, a control device 20, and a routing device 22. The clinical support subsystem 16 comprises a routing planning device 24 and a patient condition evaluation device 26. The patient transfer apparatus 12 may be a bed, a wheelchair, or an autonomous movement unit.

The route-planning device 24 is configured to provide a routing information to the routing device 22 of the patient transfer apparatus for transferring the patient transfer apparatus to a required location, such as a scan room. The route-planning device 24 may convey the routing information to the routing device 22 e.g. via a local area network connection (WLAN). The routing information may be a route map within the healthcare facility for moving the patient transfer apparatus from one location to another. The routing information may also comprise the estimation of the duration of the patient movement. In some implementations, the routing information may be based on patient, type of scan, time and modality type. For example, the routing information may comprise the estimation of the right route, time of movement and approach based on the type of scan and modality. The routing information may be displayed, visualized, e.g. on a display attached to the patient transfer apparatus, to provide required information.

The drive device 18 is configured to be controlled by the control device 20 to move the patient transfer apparatus on a first guide path according to the routing information received by the routing device 22. The control device 20 may send a control signal to drive device 18 according to the routing information received by the routing device 22. Upon receiving the control signal, the drive device 18 may cause the patient transfer apparatus 12 to move. The drive device 18 may include electric motors and wheels that are driven by the electric motors and can move in all the front, back, right, and left directions. A physical cable may be employed, e.g. a standard universal serial bus (USB) connection, to transmit the control signal and the routing information. Alternatively, the control signal and the routing information maybe transmitted wirelessly.

The in-transit patient condition assessor 14 is configured to detect at least one of a vital sign and an abnormal movement of the patient during a transit of the patient transfer apparatus 12. The in-transit patient condition assessor 14 may comprise at least one of the following: a camera 28 configured to detect an abnormal movement of the patient, a touchless sensor 30 arranged on the patient transfer apparatus and configured to detect a vital sign and/or an abnormal movement, and a patient support system 32 arranged on the patient transfer apparatus and configured to detect a vital sign. In hospitals, cameras 28 are already provided in corridors and lifts for safety or surveillance purposes. These cameras 28 may also be used for vital sign monitoring and/or abnormal movements of patients. This may be used to alert the routing device or the concerned human observer for prioritized route or movements. The patient support system 32 may comprise connected monitors, sensors, residual devices strapped on the patient transport apparatus 12.

The patient condition evaluation device 26 of the clinical decision support subsystem 16 is configured to evaluate a patient condition based on at least one of the detected vital sign and the detected abnormal movement of the patient. The patient condition may include the patient's current state, such as being good or serious. The patient condition may also include the patient short-term prognosis, for example, the patient is improving, is getting worse, or no immediate change is expected, i.e. stable. The patient condition may be evaluated and categorized in the following terms: undetermined (patient awaiting physician and/or assessment), good (vital signs are stable and within normal limits), serious (serious vital signs may be unstable and not within normal limits), and critical (critical vital signs are unstable and not within normal limits). The patient condition evaluation device 26 is capable of receiving the detected vital sign and the detected abnormal movement, and/or historical data of the patient or similar patients, and evaluating the patient condition based thereon. A predicted model may be provided to evaluate the patient's short-term prognosis. The predicted model comprises at least one of the following: a deep learning model, and a statistical model. In an example, the statistical model is an ARIMA model. In an example, the deep learning model is a LSTM model.

The route-planning device 24 is configured to update the routing information in response to the evaluated patient condition, if the evaluated patient condition meets a predefined criterion. The routing information may be updated, i.e. changed, if the evaluated patient condition meets a predefined criterion. For example, the predefined criterion may be a downgrade of the patient condition, including the current status of the patient and short-term prognosis, such as a downgrade from "good" to "serious". This usually indicates that a patient is likely to be in the intensive care unit or acute ward. If this predefined criterion is met, the patient transfer apparatus will be controlled to move the patient to the ICU for doctor's attention.

The control device 20 of the patient transfer apparatus is configured to control the drive device 18 to move the patient transfer apparatus on a second guided path according to the updated routing information during the transit of the patient transfer apparatus. The second guided path is different from the first guided path.

Accordingly, the patient transfer apparatus is aware of clinical conditions of the patient and uses the clinical conditions of the patient for routing decisions. Additionally, the system may also be adapted for non-human autonomous routing.

Optionally, the patient transfer apparatus 12 may further comprise a display 40 for visualizing the routing information as a road map to assist a user with a manual decision.

Optionally, the system 10 may further comprise a priority-planning device 34 configured to provide a priority information for triggering a control requirement for an access to a common facility within the healthcare facility such that when the patient transfer apparatus arrives at the common facility, the patient transfer apparatus is given a priority access to the common facility. For example, the priority-planning device 34, may also trigger specific control requirements, for the access to common facilities such as lift, access to corridors, so that when the patient transfer apparatus arrives, they may be at right location (floor) or reserved for them as well.

Alternatively or additionally, the priority-planning device 34 is configured to provide a priority information for determining a relative right of way for two or more patient transfer apparatuses. The priority-planning device 34 is configured to determine, according to the priority information, a first relative right of way of the patient transfer apparatus, when another patient transfer apparatus is encountered. The control device 20 of the patient transfer apparatus is configured to control the drive device 18 to move the patient transfer apparatus according to the first relative right of way. The right or way may include the right of the patient transfer apparatus to proceed with precedence over others in a particular situation or place within the healthcare facility. For example, the priority of routing may be required when a patient condition is or is becoming serious and thus the patient transfer apparatus carrying the patient has the right of way.

The priority-planning device 34 is configured to update the priority information in response to the valuated patient condition, if the evaluated patient condition meets the predefined criterion. The priority-planning device 34 is configured to determine, according to the updated priority information, a second relative right of way of the patient transfer apparatus, when another patient transfer apparatus is encountered. The control device 20 of the patient transfer apparatus is configured to control the drive device 18 to move the patient transfer apparatus according to the second relative right of way. The second relative right of way is different from the first relative right of way.

Accordingly, the patient transfer apparatus may determine the relative priority and right of way dynamically such that a patient with serious patient condition will be transferred with precedence over others and when the patient transfer apparatus arrives, they may be at right location (floor) or reserved for them as well.

The priority-planning device 34 maybe provided as a device of the patient transfer apparatus for providing the priority information based on a decision from a local distributed system. In the local distributed system, the priority-planning device 34 of each patient transfer apparatus is configured to negotiate and discuss with that of other patient transfer apparatuses according to a predefined protocol to determine each other's right or way.

Alternatively and additionally, as shown in Fig. 1, the priority-planning device 34 maybe provided as a device of the clinical decision support subsystem 16 for providing the priority information based on a decision from a centrally coordinated system. In the centrally coordinated system, the routing information of each patient transfer apparatus and the patient condition of the patient on each patient transfer apparatus are configured to be updated to determine the priority information with the priority-planning device 34 of the clinical decision support subsystem.

The system 10 may optionally comprise at least one scanner 36, and a central scan schedule subsystem 38. The system 10 may comprise multiple scanners of different modalities, such as a magnetic resonance imaging (MRI) scanner, a computed tomography (CT) scanner, a positron-emission tomography (PET) scanner, etc. The scanners may be arranged in different scan rooms.

The at least one scanner 36 is configured to perform medical imaging examinations on the patient. The central scan schedule subsystem 38 is configured to coordinate with the at least one scanner to provide a scan schedule of the patient for triggering a movement of the patient transfer apparatus towards the at least one scanner. The priority-planning device 34 of the clinical decision support subsystem is configured to determining the routing information based on the provided scan schedule. The control device 20 of the patient transfer apparatus 12 is configured to control the drive device 18 to move the patient transfer apparatus 12 on a first guided path according to the determined routing information.

The scan schedule may comprise the schedule of the patient for the scans with one or more patient scan systems. The scan schedule may determine when the next patient is due. The scan schedule may comprise at least one of the following: availability of a scanner, a scan type, a scan duration, a modality type, and a scheduling of scan slot. Based on the scan schedule, the right route, time of movement, and approach may be estimated for determining the routing information and/or priority information. Route specified preference might be based on whether the current bed is compatible to specific modality, attachment to the patients etc.

The central scan schedule subsystem 38 is configured to update the scan schedule in response to the evaluated patient condition, if the evaluated patient condition meets the predefined criterion. The route-planning device 24 and/or the priority-planning device 34 are configured to update the routing information and/or the priority information in response to the updated scan schedule. The control device 20 of the patient transfer apparatus is configured to control the drive device 18 to move the patient transfer apparatus on a second guided path according to the updated routing information and/or the updated priority information during the transit of the patient transfer apparatus. For example, the change in patients' condition results in change in priority and the route to follow. These changes are coordinated with the central scan schedule subsystem as it effects the overall scheduling of patients for the scan. For example, if the patient is not ready for the scan due to change in his clinical parameters during the transit to the scan room, immediately the system 10 routes the patient to ICU for doctor's attention and updates the same to the central scan schedule subsystem such that the reschedule of scan is performed in real time. This may improve the overall efficiency of the system in scheduling the patients for the scans and routing them autonomously.

Optionally, the central scan schedule system 10 may be configured to coordinate with the routing device 22 of individual patient transfer apparatus 12, which may allow a dynamic change in the scan schedule based on the patients' condition and a change in clinical pathways. For example, the central scan schedule subsystem 16 may obtain a local routing information of the patient transfer apparatus 12 during transit and update the scan schedule of the patient based on the local routing information of the patient transfer apparatus. The local routing information relates to the information of the surroundings of the patient transfer apparatus 12. The local routing information may comprise the position information of the patient transfer apparatus. The local routing information may also comprise traffic conditions of the surroundings of the patient transfer apparatus 12.

Fig. 2 shows a schematic diagram of a method 100 for controlling a patient transfer apparatus within a healthcare facility according to an embodiment of the invention. In step 102, a routing information is provided for transferring the patient transfer apparatus to a required location, such as a scan room or an ICU. The routing information may be a route map with further information like estimation of the duration of the patient movement. The routing information may be displayed, e.g. on a display attached to the patient transfer apparatus to provide required information.

In step 104, the patient transfer apparatus is controlled to move on a first guided path according to the provided routing information. A drive device may be provided to cause the patient transfer apparatus to move in all the front, back, right, and left directions following the routing information. The drive device may comprise electric motors and wheels that are driven by the electric motors.

In step 106, at least one of a vital sign and an abnormal movement of the patient is detected during a transit of the patient transfer apparatus. The detection maybe achieved by using an in-transit patient condition assessor. The in-transit patient condition assessor may comprise at least one of the following: a camera configured to detect an abnormal movement of the patient, a touchless sensor arranged on the patient transfer apparatus and configured to detect a vital sign and/or an abnormal movement, and a patient support system arranged on the patient transfer apparatus and configured to detect a vital sign.

In step 108, a patient condition is evaluated based on at least one of the detected vital sign and the detected abnormal movement of the patient. The patient condition may comprise the patient's current state and the patient short-term prognosis. A predicted model may be provided to evaluate the patient's short-term prognosis. The predicted model comprises at least one of the following: a deep learning model, and a statistical model. In step 110, the routing information is updated in response to the evaluated patient condition, if the evaluated patient condition meets a predefined criterion, e.g. a downgrade of the patient condition.

In step 112, the patient transfer apparatus is controlled to move on a second guided path according to the updated routing information during the transit of the patient transfer apparatus. The second guided path is different from the first path. For example, if the patient condition is downgraded from "good" to "serious", the patient transfer apparatus may change the destination and transfer the patient to the ICU instead of a scanner room.

Fig. 2 shows a schematic diagram of the method 100 according to a further embodiment of the invention. Optionally, the following further steps may be provided to determine the relative priority and right of way during routing.

In step 114, a priority information is provided for triggering a control requirement for an access to a common facility within the healthcare facility such that when the patient transfer apparatus arrives at the common facility, the patient transfer apparatus is given a priority access to the common facility.

In step 116, the priority information is provided for determining a relative right of way for two or more patient transfer apparatuses. The priority information may refer to the right of the patient transfer apparatus to proceed with precedence over others in a particular situation or place within the healthcare facility. The priority of routing may be required when a patient condition is or is becoming serious and thus the patient transfer apparatus carrying that patient has the right of way. The priority of routing may also be required to use common block points, e.g. the usage of lifts, corridors.

In step 118, a first relative right of way of the patient transfer apparatus is determined, according to the priority information, when another patient transfer apparatus is encountered.

In step 120, the patient transfer apparatus is controlled to move according to the first relative right of way.

In step 122, the priority information is updated in response to the evaluated patient condition, if the evaluated patient condition meets the predefined criterion, e.g. a downgrade of patient condition.

In step 124, a second relative right of way of the patient transfer apparatus is determined, according to the updated priority information, when another patient transfer apparatus is encountered.

In step 126, the patient transfer apparatus is controlled to move according to the second relative right of way. The second relative right of way is different from the first relative right of way.

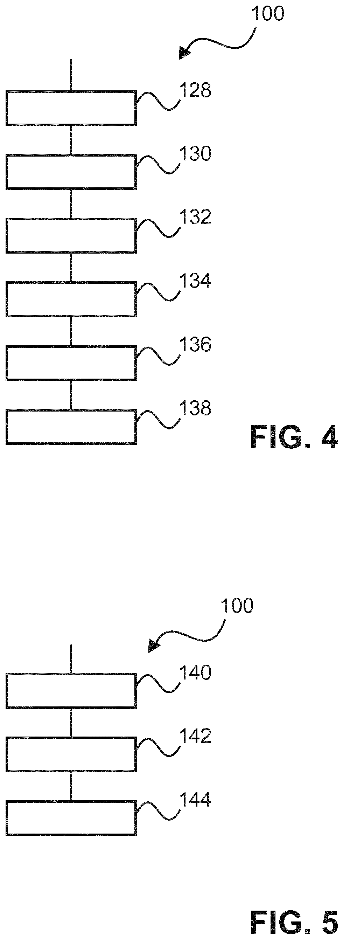

Fig. 4 shows a schematic diagram of the method according to a further embodiment of the invention. Optionally, the following further step may be provided to trigger an autonomous movement e.g. based on the scan schedule, scan duration and its type.

In step 128, a scan schedule is provided to trigger a movement of the patient transfer apparatus. The scan schedule may determine when the next patient is due. The scan schedule may comprise the availability of a scanner, scan type, scan duration, modality type and/or scheduling of scan slot.

In step 130, the routing information is determined based on the provided scan schedule. For example, the right route, time of movement and approach, can be estimated for determining the routing information and/or priority information, based on the scan schedule, e.g. the availability of the scanner, the type of scan and modality.

In step 132, the patient transfer apparatus is controlled to move on a first guided path according to the determined routing information. For example, the first guide path will lead the patient transfer apparatus to a MRI scanner room.

In step 134, the scan schedule is updated in response to the evaluated patient condition, if the evaluated patient condition meets the predefined criterion. For example, in case of a downgrade of the patient condition, the scan schedule may change, e.g. transferring the patient immediately to an ICU and rearranging the MRI scan.

In step 136, the routing information and/or the priority information is updated in response to the updated scan schedule. For example, in case of a downgrade of the patient condition, the routing information is updated such that the patient will be transfer to an ICU instead of an MRI scanner.

In step 138, the patient transfer apparatus is controlled to move on a second guided path according to the updated routing information and/or the updated priority information during the transit of the patient transfer apparatus.

Accordingly, the system, which may be an autonomous system, may be aware the availability of the scanner, scan duration, transfer time and scheduling of scans slots during autonomous movement.

Fig. 5 shows a schematic diagram of the method 100 according to a further embodiment of the invention. Optionally, the following steps may be provided.

In step 140, a local routing information of the patient transfer apparatus is obtained during transit. The local routing information may comprise position information of the patient transfer apparatus, traffic conditions of the surroundings of the patient transfer apparatus, etc.

In step 142, the scan schedule of the patient is updated based on the local routing information of the patient transfer apparatus.

This may advantageously allow a dynamic change in the scan schedule based on a change in clinical pathways in addition to the patient condition.

As a further option, in step 144, the routing information may be visualized as a road map to assist a user with a manual decision.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the system type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A method (100) for controlling a patient transfer apparatus within a healthcare facility, the method comprising the following steps:
- providing (102) a routing information for transferring the patient transfer apparatus to a required location;
- controlling (104) the patient transfer apparatus to move on a first guided path according to the provided routing information;
- detecting (106) at least one of a vital sign and an abnormal movement of the patient during a transit of the patient transfer apparatus;
- evaluating (108) a patient condition based on at least one of the detected vital sign and the detected abnormal movement of the patient;
- updating (110) the routing information in response to the evaluated patient condition, if the evaluated patient condition meets a predefined criterion; and
- controlling (112) the patient transfer apparatus to move on a second guided path according to the updated routing information during the transit of the patient transfer apparatus.

2. Method according to claim 1, further comprising the following steps:
- providing (114) a priority information for triggering a control requirement for an access to a common facility within the healthcare facility such that when the patient transfer apparatus arrives at the common facility, the patient transfer apparatus is given a priority access to the common facility; and/or
- providing (116) a priority information for determining a relative right of way for two or more patient transfer apparatuses;
- determining (118), according to the priority information, a first relative right of way of the patient transfer apparatus, when another patient transfer apparatus is encountered;
- controlling (120) the patient transfer apparatus to move according to the first relative right of way;
- updating (122) the priority information in response to the evaluated patient condition, if the evaluated patient condition meets the predefined criterion;
- determining (124), according to the updated priority information, a second relative right of way of the patient transfer apparatus, when another patient transfer apparatus is encountered; and
- controlling (126) the patient transfer apparatus to move according to the second relative right of way.

3. Method according to claim 2,
wherein the priority information is based on a decision from:
- a local distributed system; wherein in the local distributed system, each patient transfer apparatus is configured to negotiate and discuss with other patient transfer apparatuses according to a predefined protocol to determine each other's right or way; or
- a centrally coordinated system; wherein in the centrally coordinated system, the routing information of each patient transfer apparatus and the patient condition of the patient on each patient transfer apparatus are configured to be updated to determine the priority information.

4. Method according to any of claims 1 to 3, further comprising the following steps:
- providing (128) a scan schedule to trigger a movement of the patient transfer apparatus;
- determining (130) the routing information based on the provided scan schedule;
- controlling (132) the patient transfer apparatus to move on a first guided path according to the determined routing information;
- updating (134) the scan schedule in response to the evaluated patient condition, if the evaluated patient condition meets the predefined criterion;
- updating (136) the routing information and/or the priority information in response to the updated scan schedule; and
- controlling (138) the patient transfer apparatus to move on a second guided path according to the updated routing information and/or the updated priority information during the transit of the patient transfer apparatus.

5. Method according to claim 4, further comprising:
- obtaining (140) a local routing information of the patient transfer apparatus during transit; and
- updating (142) the scan schedule of the patient based on the local routing information of the patient transfer apparatus.

6. Method according to claim 4 or 5,
wherein the scan schedule comprises at least one of the following:
- availability of a scanner;
- a scan type;
- a scan duration;
- a modality type; and
- a scheduling of scan slot.

7. Method according to any of claims 1 to 6, further comprising:
- visualizing (144) the routing information as a road map to assist a user with a manual decision.

8. Method according to any of claims 1 to 7,
wherein the patient condition comprises at least one of the following:
- a current status of the patient based on an evaluation of at least one of the detected vital sign and the detected abnormal movement of the patient; and
- a patient's short-term prognosis based on an evaluation of at least one of the detected vital sign and the at least one detected abnormal movement of the patient, and historical data of the patient or similar patients.

9. Method according claim 8, wherein a predicted model is provided to evaluate the patient's short-term prognosis;
wherein the predicted model comprises at least one of the following:
- a deep learning model; and
- a statistical model.

10. A system (10) for controlling a patient transfer apparatus within a healthcare facility, comprising:
i) a patient transport apparatus (12), comprising:
- a drive device (18);
- a control device (20); and
- a routing device (22);
ii) an in-transit patient condition assessor (14); and
iii) a clinical support decision subsystem (16), comprising
- a route-planning device (24); and
- a patient condition evaluation device (26); and
wherein the route-planning device is configured to provide a routing information to the routing device of the patient transfer apparatus for transferring the patient transfer apparatus to a required location;
wherein the drive device is configured to be controlled by the control device to move the patient transfer apparatus on a first guide path according to the routing information received by the routing device;
wherein the in-transit patient condition assessor is configured to detect at least one of a vital sign and an abnormal movement of the patient during a transit of the patient transfer apparatus;
wherein the patient condition evaluation device of the clinical decision support system is configured to evaluate a patient condition based on at least one of the detected vital sign and the detected abnormal movement of the patient;
wherein the route-planning device is configured to update the routing information in response to the evaluated patient condition, if the evaluated patient condition meets a predefined criterion; and
wherein the control device of the patient transfer apparatus is configured to control the drive device to move the patient transfer apparatus on a second guided path according to the updated routing information during the transit of the patient transfer apparatus.

11. System according to claim 10,
wherein the in-transit patient condition assessor comprises at least one of the following:
- a camera (28) configured to detect an abnormal movement of the patient;
- a touchless sensor (30) arranged on the patient transfer apparatus and configured to detect a vital sign and/or an abnormal movement; and
- a patient support system (32) arranged on the patient transfer apparatus and configured to detect a vital sign.

12. System according to claim 10 and 11, further comprising:
- a priority-planning device (34) configured to provide a priority information for
i) triggering a control requirement for an access to a common facility within the healthcare facility such that when the patient transfer apparatus arrives at the common facility, the patient transfer apparatus is given a priority access to the common facility; and/or
(ii) determining a relative right of way for two or more patient transfer apparatuses;
wherein the priority-planning device is configured to determine, according to the priority information, a first relative right of way of the patient transfer apparatus, when another patient transfer apparatus is encountered;
wherein the control device of the patient transfer apparatus is configured to control the drive device to move the patient transfer apparatus according to the first relative right of way;
wherein the priority-planning device is configured to update the priority information in response to the valuated patient condition, if the evaluated patient condition meets the predefined criterion;
wherein the priority-planning device is configured to determine, according to the updated priority information, a second relative right of way of the patient transfer apparatus, when another patient transfer apparatus is encountered; and
wherein the control device of the patient transfer apparatus is configured to control the drive device to move the patient transfer apparatus according to the second relative right of way.

13. System according to any of claims 10 to 12, wherein the system further comprises:
- at least one scanner (36); and
- a central scan schedule subsystem (38);
wherein the at least one scanner is configured to perform medical imaging examinations on the patient;
wherein the central scan schedule subsystem is configured to coordinate with the at least one scanner to provide a scan schedule of the patient for triggering a movement of the patient transfer apparatus towards the at least one scanner;
wherein the priority-planning device of the clinical decision support subsystem is configured to determining the routing information based on the provided scan schedule;
wherein the control device of the patient transfer apparatus is configured to control the drive device to move the patient transfer apparatus on a first guided path according to the determined routing information;
wherein the central scan schedule subsystem is configured to update the scan schedule in response to the evaluated patient condition, if the evaluated patient condition meets the predefined criterion;
wherein the route-planning device and/or the priority-planning device are configured to update the routing information and/or the priority information in response to the updated scan schedule; and
wherein the control device of the patient transfer apparatus is configured to control the drive device to move the patient transfer apparatus on a second guided path according to the updated routing information and/or the updated priority information during the transit

14. Computer program element for controlling a system according to any of the claims 10 to 13, which, when being executed by a processing unit, is adapted to perform the method steps of any of the claims 1 to 9.

15. Computer readable medium having stored the program element of claim 14.
